(19) **Europäisches Patentamt / European Patent Office / Office européen des brevets**

(11) **EP 4 381 272 B1**

(12) **EUROPEAN PATENT SPECIFICATION**

(45) Date of publication and mention
of the grant of the patent:
**04.02.2026 Bulletin 2026/06**

(51) International Patent Classification (IPC):
**G01N 13/00** (2006.01)          **G01N 33/92** (2006.01)
**B01L 3/00** (2006.01)          **G01N 33/15** (2006.01)

(21) Application number: **22761093.8**

(22) Date of filing: **02.08.2022**

(52) Cooperative Patent Classification (CPC):
**G01N 13/00; G01N 33/15;** B01L 3/5085;
B01L 2300/165; G01N 2013/003

(86) International application number:
**PCT/EP2022/071609**

(87) International publication number:
**WO 2023/012131 (09.02.2023 Gazette 2023/06)**

(54) **MULTIWELL PLATE LIPOPHILICITY ASSAY**

TESTVERFAHREN ZUR BESTIMMUNG VON LIPOPHILIE MIT EINER MULTI-WELL-PLATTE

PLAQUE MULTIPUITS POUR L'ANALYSE DE LIPOPHILIE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **02.08.2021 EP 21189155**

(43) Date of publication of application:
**12.06.2024 Bulletin 2024/24**

(73) Proprietor: **F. Hoffmann-La Roche AG
4070 Basel (CH)**

(72) Inventors:
• **LEFÈVRE, Apolline
4070 Basel (CH)**
• **WAGNER, Bjoern
4070 Basel (CH)**

(74) Representative: **Küng, Peter
F. Hoffmann-La Roche AG
Patent Department
Grenzacherstrasse 124
4070 Basel (CH)**

(56) References cited:
**US-A1- 2006 211 121     US-B2- 7 534 338**

**Description**

The present invention provides a method for determining high, medium and low lipophilicity values of compounds.

Lipophilicity is an important molecular property in drug discovery. The exact knowledge of drug lipophilicity is useful for correlation with pharmaceutical processes such as membrane permeation, solubility, volume of distribution, metabolic stability and protein binding. Lipophilicity is expressed either by log P (octanol-water Partition coefficient for neutral species) or log D (octanol-water Distribution coefficient for charged molecules).

Usually, the lipophilicity is determined by the conventional shake-flask method (M.M. Abraham, H.S. Chadha, J.P.Dixon, and A.J. Leo. Hydrogen bonding. Part 9. The partition of solutes between water and various alcohols. Phys. Org. Chem. 7:712-716 (1994). When performed manually, this method is very time consuming (only 2-5 compounds per day). However, the number of compounds produced in drug discovery increased dramatically due to rapid analogue synthesis and combinatorial chemistry. This situation requests for a fast and efficient method for determining the lipophilicity of compounds.

EP 1 705 474 discloses an assay system for determining the lipophilicity of compounds consisting of two multiwall plates. After completion of the assay the solvent has to be removed from the plate to be able to measure the concentration of the compound in the solvent. Furthermore, the assay system has to be incubated for 12 hours until the distribution equilibrium has been reached.

Therefore, there is a requirement for a method which is fast, easy to perform and which allows the determination of lipophilicity of low soluble compounds.

Summary of the invention

In a first aspect, the present invention provides a method for determining the lipophilicity of a test compound comprising:

a) providing a multiwell plate, wherein the wells comprise at the bottom a lipophilic membrane and the bottom of the multiwell plate comprises a liquid tight barrier to seal the wells and to produce a liquid tight bottom of the multiwell plate,
b) adding a non-polar solvent to the lipophilic membranes in the wells of step a),
c) adding an aqueous solution comprising the test compound to the wells of step b, and
d) determining the quantity of the test compound in the aqueous solution after a distribution equilibrium has been reached.

In an embodiment of the method for determining the lipophilicity of a test compound, the non-polar solvent is octanol.

In an embodiment of the method for determining the lipophilicity of a test compound, the lipophilic membrane is selected from the group consisting of polyvinylidene fluoride (PVDF), polytetrafluoroethylene (PTFE), cyclic olefin copolymer (COC), polypropylene (PP) or polycarbonate (PC), preferably a PVDF membrane.

In an embodiment of the method for determining the lipophilicity of a test compound, the multiwell plate is a 96 multiwell plate, preferably a MultiScreen hydrophobic Immobilon P PVDF membrane plate welded at the bottom with a liquid tight foil, preferably a heat sealing foil.

In an embodiment of the method for determining the lipophilicity of a test compound, the method comprises the additional step e) of calculating the lipophilicity value log D of the test compound.

In a second aspect not forming part of the present invention, the disclosure provides a multiwell plate for use in a method for determining the lipophilicity of a test compound, wherein the wells comprise a lipophilic membrane at the bottom of the wells and the bottom of the multiwell plate comprises a liquid tight barrier.

In an example of the multiwell plate for use in a method for determining the lipophilicity of a test compound, the lipophilic membrane is selected from the group consisting of polyvinylidene fluoride (PVDF), polytetrafluoroethylene (PTFE), cyclic olefin copolymer (COC), polypropylene (PP) or polycarbonate (PC).

In an example of the multiwell plate for use in a method for determining the lipophilicity of a test compound, the liquid tight barrier is a liquid tight foil, preferably a heat sealing foil.

In an example of the multiwell plate for use in a method for determining the lipophilicity of a test compound, the lipophilic membrane is a PVDF membrane.

In an example of the multiwell plate for use in a method for determining the lipophilicity of a test compound, the multiwell plate is a 96 - well plate.

In an example of the multiwell plate for use in a method for determining the lipophilicity of a test compound, the multiwell plate is a MultiScreen hydrophobic Immobilon P PVDF membrane plate, wherein the bottom of the plate is welded with a liquid tight foil, preferably a heat sealing foil.

In an example of the multiwell plate for use in a method for determining the lipophilicity of a test compound, the method for determining the lipophilicity of a compound is a method according to the method of the present invention.

In a third aspect not forming part of the present invention, the disclosure provides a MultiScreen hydrophobic Immobilon P PVDF membrane plate, wherein the bottom of the plate is welded with a heat sealing foil.

Detailed description of the invention

The test compound may be any chemical or biological compound. The test compound may be for example an organic compound, a protein, a peptide or a nucleic acid. An organic compound may include also organic-inorganic molecules. The term organic-inorganic molecule as used herein refers to an organic molecule in which at least one inorganic atom is bound to a carbon atom. An inorganic atom may i.e. a metal atom such as i.e. silicon (Si) or germanium (Organometallics, i.e. Si or Ge bioisoester of organic molecules).

The test compound may be solid or liquid. The test compound is dissolved in an aqueous solution. The test compound may a lipophilic compound or a hydrophilic compound.

The term "multiwell plate" as used herein refers to a plate with multiple wells used as small test tubes. Multiwell plates are commercially available as 24 well, 48 well, 96 well and 384 well plates. The multiwell plate can be made of a variety of materials such as e.g. polystyrene, polypropylene and acrylonitrile butadiene styrene (ABS).

The liquid tight barrier is preferably a liquid tight foil, preferably a heat sealing foil. When heat sealing, the sealing material is first placed in position on the plate. Heat is applied evenly for several seconds, resulting in binding of the sealing material to the plate to achieve a complete seal. Heat sealing foils are commercially available from e.g. Thermo Fisher scientific.

The term "non-polar solvent" as used herein refers to a hydrophobic solvent. Non-polar solvents are immiscible, or hardly miscible with polar solvents such as for example water. A lipophilic compound has usually the tendency to be more soluble in a non-polar solvent than in a polar solvent. The dielectric constant of a non-polar solvent is usually lower than that of water. Examples of a hydrophobic solvent are organic solvents such as i.e. octanol or aliphatic hydrocarbons (dodecan, hexadecane or halogenized hydrocarbons).

The aqueous solution may be for example a hydrophilic buffer solution which could consist of a buffer salt (i.e. aqueous solutions of phosphate or TAPSO salts buffered at pH 7.4) in water with high buffer capacity within the pH range of interest. The pH of interest may be in the range between pH 0 to 14, preferably the pH is about 7.4.

The term "distribution equilibrium" as used herein refers to the equilibrium of distribution between the aqueous solution comprising the test compound and the non-polar solvent used to impregnate the lipophilic membrane. Preferably, the distribution equilibrium is achieved between 0.1 - 24 h, more preferably it is achieved within 2h.

The term "lipophilic membrane" as used herein refers to a membrane for non-polar solvent. Such membranes may be formed as a mesh out the lipophilic material or a layer with pores. Preferably, the pore size or mesh size is in the range between 0.01 - 100 $\mu$m. The lipophilic membrane material comprises but is not limited to polyvinylidene fluoride (PVDF), polytetrafluoroethylene (PTFE), cyclic olefin copolymer (COC), polypropylene (PP) or polycarbonate (PC).

The lipophilic membrane, preferably a PVDF membrane, may be impregnated by applying the non-polar solvent to the membrane whereby the membrane is able to absorb the solvent completely. The solvent may be applied i.e. by a dispenser. Further methods are known in the art such as for example robotic liquid handling system, which allows to dispense 0.1 $\mu$l - 50 $\mu$l/cm$^2$ of the organic modifier on the surface of the membrane.

The quantity of the test compound in the aqueous solution may be determined by methods comprising but not limited to the group consisting of UV- and/or mass spectroscopy, capillary electrophoresis (CE) and high pressure liquid chromatography (HPLC).

A lipophilic test compound may be for example polycyclic aromatic or aliphatic hydrocarbons, fat soluble vitamins, hydrophobic drugs like fungicides, halogen-containing aromatic or aliphatic hydrocarbons, nitrogen and oxygen containing aromatic or aliphatic hydrocarbons.

The non-polar solvent is immiscible or hardly miscible with the aqueous solution comprising the test compound. The preferred non-polar solvent is octanol (octan-1-ol). The preferred aqueous solution is water or a buffer.

The test compound may be solid or liquid. The test compound may be dissolved in a suitable solvent as for example DMSO (dimethyl sulfoxide). A suitable solvent for a hydrophilic compound is preferably a polar solvent; a suitable solvent for a lipophilic compound is preferably a non polar solvent.

The method of the present invention offers the following advantages over the method disclosed in EP 1 705 474: no separation of the solvents A and B is necessary to be able to measure the concentration of the test compound in solvent B. The multiwell plate containing the aqueous solution comprising the test compound can be directly inserted in an appropriate device to measure the concentration of the test compound after the distribution equilibrium has been reached. Furthermore, the incubation time to reach a distribution equilibrium is about 1.5 hours compared to 12 hours in the method of EP 1 705 474.

Brief description of the figures:

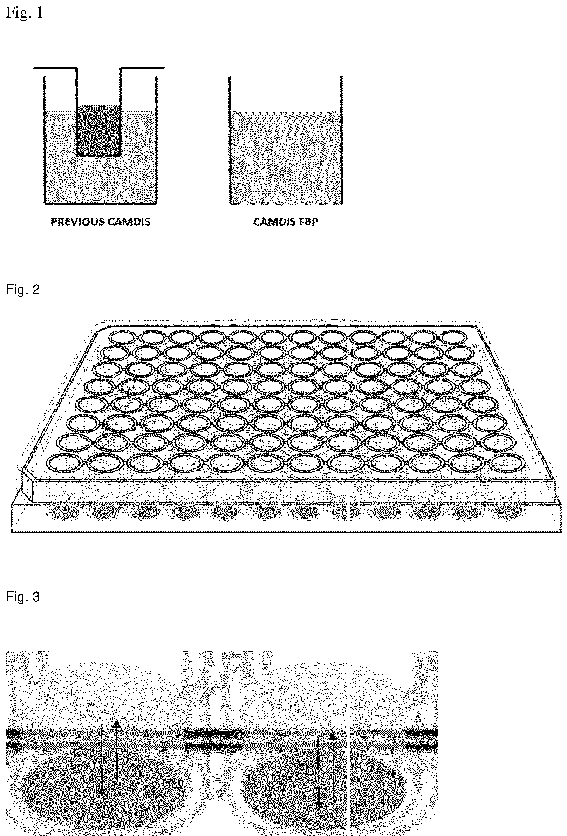

Fig. 1: Schemes of the different types of wells used for the previous CAMDIS (see EP 1705474) and the new CAMDIS Filter Bottom Plate (FBP). In the previous CAMDIS, octanol (in red) is poured in a DIFI tube that is put into contact with the aqueous solution. In CAMDIS FBP, the filter is directly coated with octanol.

Fig. 2 shows an exemplary 96 - multiwell plate with a PVDF membrane (red) sealing the wells at the bottom. The bottom of such a plate is welded with a foil to have liquid tight wells. Preferred foils are heat sealing foils. The resulting liquid tight 96 multiwell plate can be used in the lipophilicity method of the present invention.

Fig. 3 shows a detailed view of the wells of a multiwell plate with sealed bottom. The PVDF Membrane on the bottom of each well is used as carrier for octanol (red). Aqueous sample solutions cover the PVDF membrane (blue). The bottom of the plate is welded with a heat sealing foil. The same plate layout as for CAMDIS.

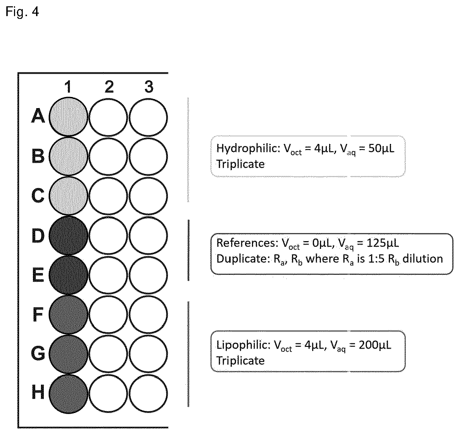

Fig. 4: Assay plate format for CAMDIS FBP method. LogD values in hydrophilic and lipophilic conditions are measured in triplicates.

Examples

Carrier Mediated Distribution System (CAMDIS) Filter Bottom Plate

The method of the present invention retains the principle used in the classical shake flask method as in the previous CAMDIS (see Wagner et al.: Eur J Pharm Sci. 2015 Feb 20; 68:68-77), namely the analysis of the concentration ratio of a drug distributed in the two phases (aqueous buffer and an organic solvent, typically octanol). The principal difference between the set-up of the present CAMDIS method and the CAMDIS method disclosed in EP 1 705474 is the use of a filter bottom plate (e.g. Millipore Multiscreen filter plate, MSIPN45) instead of the two previously used plates. The filter of this new plate is made of the same material as the DIFI tube, namely hydrophobic PVDF. This hydrophobic material was used in order to prevent the octanol phase to mix with the aqueous phase. The membrane can be coated with octanol and then covered by the aqueous solution without mixing the two phases.

Incubation time

The main advantage of the method of the present invention is that the exchange surface between the two phases is greater than in the previous set-up due to the use of a new plate. Indeed, the DIFI tubes used in the last CAMDIS version had a diameter of 2.3mm while new filter bottom plate has a diameter of 6.6mm. It is thus expected that the incubation time required to reach the distribution's equilibrium would be shorter in CAMDIS Filter Bottom Plate as a greater surface is available for exchange between the two phases.

LC-MS/UV was used to measure the UV absorption of three standard compounds, covering a broad range of logD values. All compounds were directly ordered as Stock solutions (10 mM) from the Roche internal library and successfully passed the quality controls prior to use. The analyses were made at different time points (different incubation times) and compared to the logD values determined by the previous CAMDIS method. The experiment is extensively described in the Appendix section 5.1.3. Based on the results displayed in Figure 3, an incubation time of 90 minutes while shaking at 1'000rpm was recommended to obtain the most accurate logD values for both phase volume ratios. Further experiments should be performed to measure the evaporation effect on the compound's concentration, particularly under hydrophilic conditions.

CAMDIS Filter Bottom Plate Protocol (CAMDIS FBP)

Prior to the experiment, the bottom of the filter bottom plate was welded with a heat sealing foil to prevent leaking or evaporation of the octanol phase. As in the previous CAMDIS version, octanol and phosphate buffer (25 mM, pH 7.4) were mutually saturated at room temperature. 14 $\mu$L of drug as DMSO stock solution (10 mM) was introduced into 1200 $\mu$L of aqueous buffer. Solutions were filtered in the same fashion as previous CAMDIS and a second dilution was performed to reach a final volume of 1400$\mu$L. The filter bottom plate was coated with 4 $\mu$L of octanol using the automatic liquid dispenser according to the plate layout displayed in Figure 4. Aliquots of 50 or 200 $\mu$L of the filtrate were transferred into the precoated plate. References Rb consisted of 150 $\mu$L of aqueous buffer in a well not coated with octanol. A 1:5 dilution, referred to as Ra, of the Rb reference was also performed. Plates were sealed and shaken at 1000rpm, 21 "C for 90 minutes. The equilibrium aqueous drug concentration was analyzed by LCMS/UV. Several injections into Rb were performed with 4 different injection volumes(1, 2, 3 and 4 $\mu$L). Those were used to make a calibration curve of the peak area in function of the injected volume, required for analysis with MS. Indeed, when using UV data, the Beer Lambert law states that absorption is directly proportional to the concentration of the compound such that Eq. 2 can be directly used by replacing the concentration by the UV peak area, but this is not the case for MS. Also, UV detection has a wide range of linearity, which is independent from the compound properties. In contrast, the used MS-method had a limited linearity range in terms of its readout depending strongly on the compound characteristics. Therefore, calibration of the peak area versus concentration was needed for each compound when using MS data. The calibration was done by using the four injections of Rb and the Ra reference, to fit a 2nd order polynomial function. This equation allowed to determine the injected volume as a function of the MS peak area (proportional to the amount of drug) and then the injected volume was used instead of the amount of substance to compute the logD as follows:

$$logD_{MS} = log10\left(\frac{V_{inj,R_{b4}} - V_{inj,sample}}{V_{inj,sample}} \; x \; \frac{V_{water}}{V_{oct}}\right)$$

Assay validation

The same set of drugs (Wagner et al. Eur J Pharm Sci, 68:68-77, 2015.) used in the last version of CAMDIS was used in order to validate the new experimental set-up of CAMDIS Filter Bottom Plate (see table 1). The set was compiled with 52 drugs with known logD values determined with the shake flask method or the miniaturized shake flask method at pH 7.4. The average literature value was taken and compared with the average logD value obtained from the new CAMDIS set-up for each compound. The standard deviation across the literature was also computed as an additional quality parameter and was considerable for some compounds.

The measured compounds along with their literature and CAMDIS Filter Bottom Plate logD values and standard deviations are listed in Table 1. Using dexamethasone as standard, the octanol volume was adjusted to compensate for the error of the automatic liquid dispenser. The same correction volume was then applied to all the compounds. LogD values were not able to be computed for three compounds (Cimetidine, Disopyramide and Erythromycin) due to insufficient data quality. The previous CAMDIS method was not able to measure those logD either and the literature standard deviation for those compounds was quite high.

Conclusion

The results in this section demonstrate that the new CAMDIS Filter Bottom Plate method is a promising new method for the measurement of logD values. Compared to the previous CAMDIS method, the handling is simplified thanks to the use of only one filter bottom plate instead of the sandwich plates. The new CAMDIS Filter Bottom Plate method allows an eight times faster determination of logD values compared to the CAMDIS Pool method and yields values that are in excellent agreement with literature shake flask values as well as with previous CAMDIS values. The method allows to measure logD values in a broad range, from -0.2 to 4, depending on the readout of each compound, which is the typical range for molecules in the drug discovery scope. Compared to the shake flask method and like the previous CAMDIS version, the method reduces the time required to obtain logD values by deleting the phase separation process. Indeed, in CAMDIS, the two phases, octanol and aqueous buffer, do not need to be separated to determine the drug concentration as opposed to the shake flask method. Compared to the previous CAMDIS Pool version, the process is even faster due to the increase of the surface available for the compound's exchange across the two phases, which reduces the incubation time from 12 hours to 90 minutes.

Table 1. Comparison between CAMDIS FBP log D (pH 7.4) and literature shake flask log D values (pH 7.4).

| Compound | Literature [1] | | CAMDIS FBP | | |
|---|---|---|---|---|---|
| | LogD | SD | LogD | SD* | N |
| Albendazole | 3.29 | - | 3.41 | 0.01 | 6 |
| Alprenolol | 0.88 | 0.13 | 1.03 | 0.06 | 3 |
| Amitriptyline | 2.79 | 0.34 | 2.87 | - | 2 |
| Antipyrine | 0.24 | 0.01 | 0.40 | 0.01 | 3 |
| Buspirone | 2.14 | - | 2.26 | 0.04 | 3 |
| Caffeine | -0.04 | 0.06 | 0.06 | 0.04 | 6 |
| Carbamazepine | 1.65 | - | 1.59 | 0.03 | 6 |
| Chloramphenicol | 1.12 | 0.04 | 1.08 | 0.02 | 6 |
| Chlorpromazine | 3.16 | 0.11 | 3.26 | 0.03 | 3 |
| Chlorthalidone | 0.85 | - | 0.90 | 0.02 | 6 |
| Clomipramine | 3.28 | 0.06 | 3.46 | 0.02 | 3 |
| Coumarin | 1.42 | 0.04 | 1.57 | 0.00 | 3 |
| Desipramine | 1.41 | 0.09 | 1.30 | 0.02 | 3 |
| Dexamethason | 1.82 | 0.11 | 1.82 | 0.01 | 6 |
| Diazepam | 2.74 | 0.1 | 2.75 | 0.02 | 3 |
| Diclofenac | 1.14 | 0.05 | 1.12 | 0.03 | 6 |
| Fentanyl | 2.86 | - | 2.99 | 0.02 | 6 |
| Fluoxetine | 1.88 | 0.08 | 2.01 | 0.02 | 3 |
| Flurbiprofen | 0.91 | - | 0.80 | 0.04 | 6 |
| Glyburide | 2.19 | - | 2.06 | 0.01 | 3 |
| Griseofulvin | 2.18 | - | 2.19 | 0.05 | 6 |
| Hydrochlorothiazide | -0.12 | - | -0.27 | - | 2 |
| Hydrocortisone | 1.57 | 0.06 | 1.53 | 0.03 | 6 |
| Ibuprofen | 1.27 | 0.34 | 0.97 | 0.01 | 3 |
| Imipramine | 2.51 | 0.02 | 2.30 | 0.05 | 6 |
| Indomethacin | 0.76 | 0.22 | 0.87 | 0.04 | 6 |
| Ketoprofen | -0.23 | 0.04 | -0.12 | 0.05 | 3 |
| Labetalol | 1.09 | - | 1.08 | 0.02 | 6 |
| Lidocaine | 1.61 | 0.31 | 1.66 | 0.00 | 6 |

| Compound | Literature [1] | | CAMDIS FBP | | |
|---|---|---|---|---|---|
| | LogD | SD | LogD | SD* | N |
| Meprobamate | 0.7 | - | 0.76 | 0.02 | 3 |
| Mesoridazine | 1.81 | - | 1.74 | 0.01 | 3 |
| Metoclopramide | 0.53 | 0.1 | 0.61 | 0.02 | 3 |
| Metoprolol | -0.17 | 0.2 | 0.06 | 0.03 | 3 |
| Metronidazole | -0.07 | 0.06 | -0.22 | 0.05 | 3 |
| Naproxen | 0.32 | 0.02 | 0.18 | 0.05 | 5 |
| Nimodipine | 4.18 | - | 3.95 | 0.01 | 3 |
| Paracetamol | 0.3 | - | 0.24 | 0.02 | 6 |
| Phenytoin | 2.23 | 0.38 | 2.32 | 0.04 | 6 |
| Prednisolone | 1.45 | 0.04 | 1.53 | 0.05 | 6 |
| Primidone | 0.91 | - | 0.74 | 0.04 | 6 |
| Progesterone | 3.76 | 0.19 | 3.62 | 0.03 | 6 |
| Propranolol | 1.21 | 0.16 | 1.14 | 0.01 | 3 |
| Temazepam | 1.98 | 0.11 | 2.06 | 0.03 | 6 |
| Testosterone | 3.23 | 0.16 | 3.12 | 0.01 | 3 |
| Theophyline | -0.02 | - | -0.16 | 0.06 | 3 |
| Triflupromazine | 3.39 | - | 3.49 | 0.05 | 3 |
| Trimethoprim | 0.64 | - | 0.54 | 0.03 | 3 |
| Warfarin | 1.12 | 0.11 | 0.87 | 0.01 | 6 |

\* Standard deviation is not computed when only two logD values are available

[1] Wagner, B. et all: Carrier Mediated Distribution System (CAMDIS): A new approach for the measurement of octanol/water distribution coefficients, European Journal of Pharmaceutical Sciences 68 (2015) 68–77 "

## Claims

1. A method for determining the lipophilicity of a test compound comprising:

    a) providing a multiwell plate, wherein the wells comprise at the bottom a lipophilic membrane and the bottom of the multiwell plate comprises a liquid tight barrier to seal the wells and to produce a liquid tight bottom of the multiwell plate,
    b) adding a non-polar solvent to the lipophilic membranes in the wells of step a),
    c) adding an aqueous solution comprising the test compound to the wells of step b, and
    d) determining the quantity of the test compound in the aqueous solution after a distribution equilibrium has been reached.

2. The method of claim 1, wherein the non-polar solvent is octanol.

3. The method of claim 1 or 2, wherein the lipophilic membrane is selected from the group consisting of polyvinylidene fluoride (PVDF), polytetrafluoroethylene (PTFE), cyclic olefin copolymer (COC), polypropylene (PP) or polycarbonate (PC), preferably a PVDF membrane.

4. The method of claims 1 - 3, wherein the multiwell plate is a 96 multiwell plate welded at the bottom with a liquid tight foil, preferably a heat sealing foil.

5. The method of claims 1 - 4 comprising the additional step e) of calculating the lipophilicity value log D of the test compound.

# EP 4 381 272 B1

**Patentansprüche**

1. Verfahren zum Bestimmen der Lipophilie einer Testverbindung, umfassend:

   a) Bereitstellen einer Multi-Well-Platte, wobei die Wells am Boden eine lipophile Membran umfassen und der Boden der Multi-Well-Platte eine flüssigkeitsdichte Barriere umfasst, um die Wells abzudichten und einen flüssigkeitsdichten Boden der Multi-Well-Platte zu erzeugen,
   b) Zugeben eines unpolaren Lösungsmittels zu den lipophilen Membranen in den Wells von Schritt a),
   c) Zugeben einer wässrigen Lösung, die die Testverbindung umfasst, in die Wells von Schritt b und
   d) Bestimmen der Menge der Testverbindung in der wässrigen Lösung, nachdem ein Verteilungsgleichgewicht erreicht wurde.

2. Verfahren nach Anspruch 1, wobei das unpolare Lösungsmittel Octanol ist.

3. Verfahren nach Anspruch 1 oder 2, wobei die lipophile Membran ausgewählt ist aus der Gruppe bestehend aus Polyvinylidenfluorid (PVDF), Polytetrafluorethylen (PTFE), cyclischem Olefincopolymer (COC), Polypropylen (PP) oder Polycarbonat (PC), vorzugsweise einer PVDF-Membran.

4. Verfahren nach den Ansprüchen 1-3, wobei die Multi-Well-Platte eine 96-Multi-Well-Platte ist, die am Boden mit einer flüssigkeitsdichten Folie, vorzugsweise einer Heißsiegelfolie, verschweißt ist.

5. Verfahren nach den Ansprüchen 1-4, umfassend den zusätzlichen Schritt e) des Berechnens des Lipophiliewerts log D der Testverbindung.

**Revendications**

1. Procédé de détermination de la lipophilie d'un composé de test comprenant :

   a) la fourniture d'une plaque multipuits, dans lequel les puits comprennent au fond une membrane lipophile et le fond de la plaque multipuits comprend une barrière étanche aux liquides pour sceller les puits et pour produire un fond étanche aux liquides de la plaque multipuits,
   b) l'ajout d'un solvant non polaire aux membranes lipophiles dans les puits de l'étape a),
   c) l'ajout d'une solution aqueuse comprenant le composé de test aux puits de l'étape b, et
   d) la détermination de la quantité du composé de test dans la solution aqueuse après qu'un équilibre de distribution a été atteint.

2. Procédé selon la revendication 1, dans lequel le solvant non polaire est l'octanol.

3. Procédé selon la revendication 1 ou 2, dans lequel la membrane lipophile est choisie dans le groupe constitué par le fluorure de polyvinylidène (PVDF), le polytétrafluoroéthylène (PTFE), un copolymère d'oléfines cyclique (COC), le polypropylène (PP) ou le polycarbonate (PC), de préférence une membrane en PVDF.

4. Procédé selon les revendications 1 à 3, dans lequel la plaque multipuits est une plaque à 96 puits soudée au fond avec un film étanche aux liquides, de préférence un film thermoscellable.

5. Procédé selon les revendications 1 à 4 comprenant l'étape e) supplémentaire de calcul de la valeur de lipophilie log D du composé de test.

Fig. 1

PREVIOUS CAMDIS          CAMDIS FBP

Fig. 2

Fig. 3

Fig. 4

Hydrophilic: $V_{oct} = 4\mu L$, $V_{aq} = 50\mu L$
Triplicate

References: $V_{oct} = 0\mu L$, $V_{aq} = 125\mu L$
Duplicate: $R_a$, $R_b$ where $R_a$ is 1:5 $R_b$ dilution

Lipophilic: $V_{oct} = 4\mu L$, $V_{aq} = 200\mu L$
Triplicate

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 1705474 A

**Non-patent literature cited in the description**

- **M.M. ABRAHAM ; H.S. CHADHA ; J.P.DIXON ; A.J. LEO**. Hydrogen bonding. Part 9. The partition of solutes between water and various alcohols. *Phys. Org. Chem.*, 1994, vol. 7, 712-716
- **WAGNER et al.** *Eur J Pharm Sci.*, 20 February 2015, vol. 68, 68-77
- **WAGNER et al.** *Eur J Pharm Sci*, 2015, vol. 68, 68-77